# EUROPEAN PATENT APPLICATION

(11) **EP 4 629 252 A1**
(43) Date of publication of application: **08.10.2025**
(21) Application number: 23897148.5
(22) Date of filing: 23.08.2023
(51) Int. Cl.: G16C 60/00, G16C 20/10

(54) **PREDICTION SYSTEM, PREDICTION DEVICE, AND PREDICTION METHOD**

(30) Priority: 30.11.2022 JP 2022191258
(71) Applicant: Hitachi, Ltd., Tokyo 100-8280 (JP)
(72) Inventor: MURAKAMI Hitomi, Tokyo 100-8280 (JP); MORI Shunsuke, Tokyo 100-8280 (JP)
(74) Representative: Mewburn Ellis LLP
(86) International application number: PCT/JP2023/030333
(87) International publication number: WO 2024/116488

(57) **Abstract**

Provided is a prediction system capable of predicting the reaction percentage of molecules that form a resin. To solve this problem, a prediction system (100) comprises: a prediction device (20) which predicts a reaction percentage of reactive groups in a reaction between the reactive groups, the reactive groups included in monomers being molecules that form a resin. The prediction device (20) includes: a DB (222) in which a first indicator of each of the monomers related to the activation energy defined by the Arrhenius equation, a second indicator of each of the monomers related to the frequency factor defined by the Arrhenius equation, and the reaction percentage are associated with one another; and a prediction unit (214) which predicts the reaction percentage from the first indicators and the second indicators of the monomers determined based on molecule information on the monomers, and from the DB (222).

## Description

### TECHNICAL FIELD

The present disclosure relates to a prediction system, a prediction device, and a prediction method.

### BACKGROUND ART

Properties of resins vary by the percentages of reactions such as the reaction that synthesizes the resin from monomers serving as raw material molecules and a reaction that forms or dissociates a bond(s) that binds an additive and the resin. Thus, in the materials design of resins, it is preferable to predict how much reaction percentage the monomers to be used have. Also, for monomers that form resins of the same type (e.g., polyimides), there can be many substituent types and bonding positions. A difference in structure may change the ease with which the reaction progresses. Hence, each time the composition is changed, it is preferable to conduct an experimental study and evaluate the degree of progress of the reaction and performance.

To determine the composition of a resin and a process therefor for the purpose of evaluation, the resin can be actually manufactured by various processes to evaluate the reaction percentage. In this case, fitting can be performed with the Arrhenius equation or the like to practice trial and error for searching for the optimal composition and process. Such an approach, however, involves a high volume of experiments to be performed by operators and a high cost for obtaining materials.

In recent years, the utilization of digital technologies such as computer simulation and machine learning using databases or the like has become increasingly prevalent in materials design. The abstract of Patent Literature 1 discloses that "[a] reaction mechanism generation method comprises the steps of: carrying out the molecular dynamics calculation on atoms constituting each molecule in a reaction system in every time step; when a chemical reaction occurs in the reaction system before and after the time step, identifying a reaction molecule that contributes to the chemical reaction and a generated molecule; constituting an elementary reaction constituted by the reaction molecule and the generated molecule which are related to each other on the basis of the atom-level relation between the reaction molecule and the generated molecule; and calculating a reaction rate constant of the aboveconstituted elementary reaction".

### CITATION LIST

### Patent Literature

Patent Literature 1: WO2016/133002

### SUMMARY OF INVENTION

### Technical Problem

The technique described in Patent Literature 1 is a technique for calculating a reaction rate constant, not a technique for predicting the reaction percentage of molecules that form a resin in a reaction (polymerization) between the molecules.

A problem to be solved by the present disclosure is to provide a prediction system, a prediction device, and a prediction method which are capable of predicting the reaction percentage of molecules that form a resin.

### Solution to Problem

A prediction system of the present disclosure is a prediction device which predicts a reaction percentage of reactive groups in a reaction between the reactive groups, the reactive groups included in monomers being molecules that form a resin, wherein the prediction device includes a database in which a first indicator of each of the monomers related to the activation energy defined by the Arrhenius equation, a second indicator of each of the monomers related to the frequency factor defined by the Arrhenius equation, and the reaction percentage are associated with one another, and a prediction unit which predicts the reaction percentage from the first indicators and the second indicators of the monomers determined based on molecule information on the monomers, and from the database. Other solving means will be described later in modes for carrying out the invention.

### Advantageous Effect of Invention

According to the present disclosure, it is possible to provide a prediction system, a prediction device, and a prediction method which are capable of predicting the reaction percentage of molecules that form a resin.

### BRIEF DESCRIPTION OF DRAWINGS

FIG. 1 is a block diagram of a prediction device of the present disclosure.
FIG. 2 is a schematic view of an input screen according to one embodiment.
FIG. 3 is a block diagram showing a hardware configuration of a prediction device according to one embodiment.
FIG. 4 is a schematic diagram showing a hierarchical structure according to one embodiment intended to extract features related to the frequency factor.
FIG. 5 is a schematic diagram of a molecular property database according to one embodiment.
FIG. 6 is a flowchart showing a method of constructing the molecular property database according to one embodiment.
FIG. 7 is a schematic diagram of a second structure database according to one embodiment.
FIG. 8 is a schematic diagram showing a hierarchical structure according to one embodiment intended to extract features related to the activation energy.
FIG. 9 is a schematic diagram of a reaction path database according to one embodiment.
FIG. 10 is a flowchart showing a method of constructing the reaction path database according to one embodiment.
FIG. 11 is a schematic diagram of a first structure database according to one embodiment.
FIG. 12 is a schematic diagram of a prediction model database according to one embodiment.
FIG. 13 is a graph comparing predicted values and measured values when prediction model are created in one embodiment.
FIG. 14 is a flowchart showing a prediction method of the present disclosure.

### DESCRIPTION OF EMBODIMENTS

Modes for carrying out the present disclosure (referred to as "embodiments") will be described below with reference to the drawings. In the following description of one embodiment, other embodiments applicable to the one embodiment will also be described as appropriate. The present disclosure is not limited to the following one embodiment, and it is possible to combine different embodiments or modify the present disclosure as desired to such an extent as not to significantly impair its advantageous effect. Also, identical members will be denoted by the same reference sign, and overlapping description will be omitted. Moreover, elements having the same function will be given the same name. The contents shown in the drawings are merely schematic. For convenience of illustration, changes may be made to the actual configuration or the illustration of some members in drawings may be omitted or modified in others drawings to such an extent as not to significantly impair the advantageous effect of the present disclosure. Also, it is not necessarily essential to include all of the components in a single embodiment.

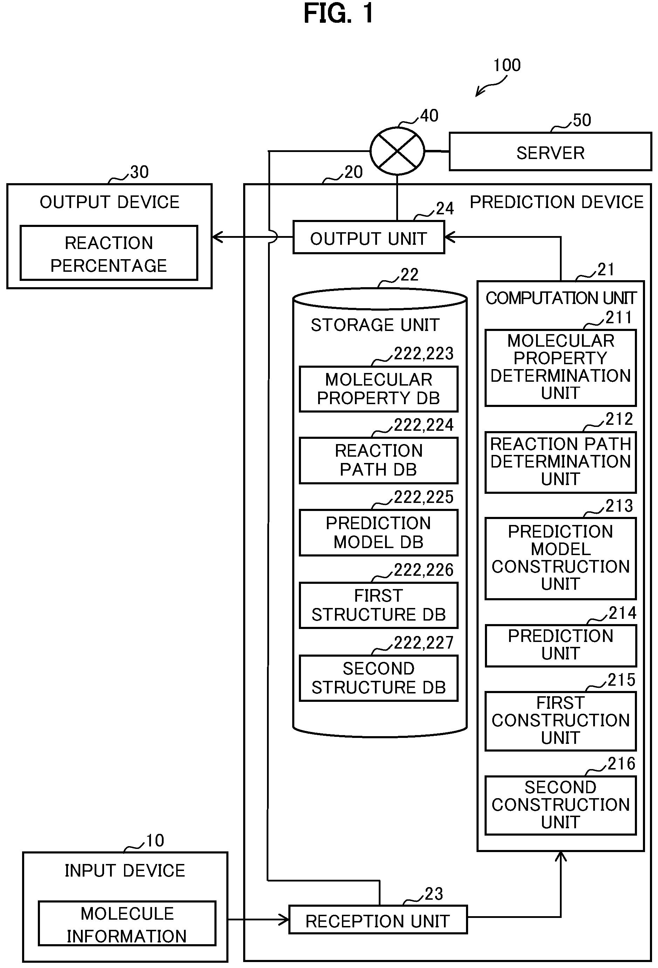

FIG. 1 is a block diagram of a prediction system 100 of the present disclosure. The prediction system 100 includes an input device 10, a prediction device 20, and an output device 30. The prediction system 100 and the prediction device 20 are intended to predict the reaction percentage of reactive groups included in monomers being molecules that form a resin in a reaction between the reactive groups. The prediction is executed by using, for example, a database of parameters that differ from one raw material to another, such as the activation energy and the frequency factor (each of which will be described later). Besides monomers (raw materials) being molecules that form a resin, the molecules mentioned here may be oligomers each being a plurality of monomers bound to each other. Molecule information is information on a monomer and is, for example, the type of the monomer (molecular formula, rational formula, etc.).

The reaction percentage is the reaction percentage (reaction ratio) of reactive groups included in monomers in a reaction between the reactive groups, as mentioned above. A reactive group is a reactive functional group that is involved in a formation reaction (polymerization) of a resin (reactive site), and this functional group includes double bonds, triple bonds, and the like. In the present disclosure, the reaction percentage is defined by the following equation. Reaction percentage (%) = (Number of reacted reactive groups/Number of reactive groups at the start of the reaction) × 100

For example, in the case of producing polyethylene (resin) from ethylene (raw material, monomer), the double bond (reactive group) forming ethylene turns into a single bond, allowing polymerization of ethylene. Thus, in the case where 10 ethylene molecules are present, the number of reactive groups at the start of the reaction is 10. Then, if, for example, nine double bonds react, the number of reacted functional groups is nine, so that the reaction percentage is (9/10) × 100 = 90%. Note that the polymerization is not limited to a polymerization that occurs as a result of a double bond or a triple bond turning into a single bond or the like, and may be any polymerization, such as condensation polymerization or ring-opening polymerization, for example.

The input device 10, the prediction device 20, and the output device 30 are connected to one another. The prediction device 20 (in particular, a reception unit 23 and an output unit 24) is connected to an external server 50 through a network 40. The prediction device 20 may have a function of receiving inputs from and sending outputs to the server 50 and the external network 40. In this case, an input device and an output device installed at a remote location (neither of which is shown) may be used in place of the input device 10 and the output device 30. Information in a storage unit 22 may be updated based on information obtained externally via the server 50. Note that it is not necessary for the prediction device 20 to be connected to the network 40 and the server 50.

The input device 10 includes, for example, at least one of a mouse, a keyboard, a touch panel, a display, a monitor, or the like, neither of which is shown. The output device 30 displays predicted reaction percentages and so on. The output device 30 is a display device, such as a display, a printer, or the like, for example.

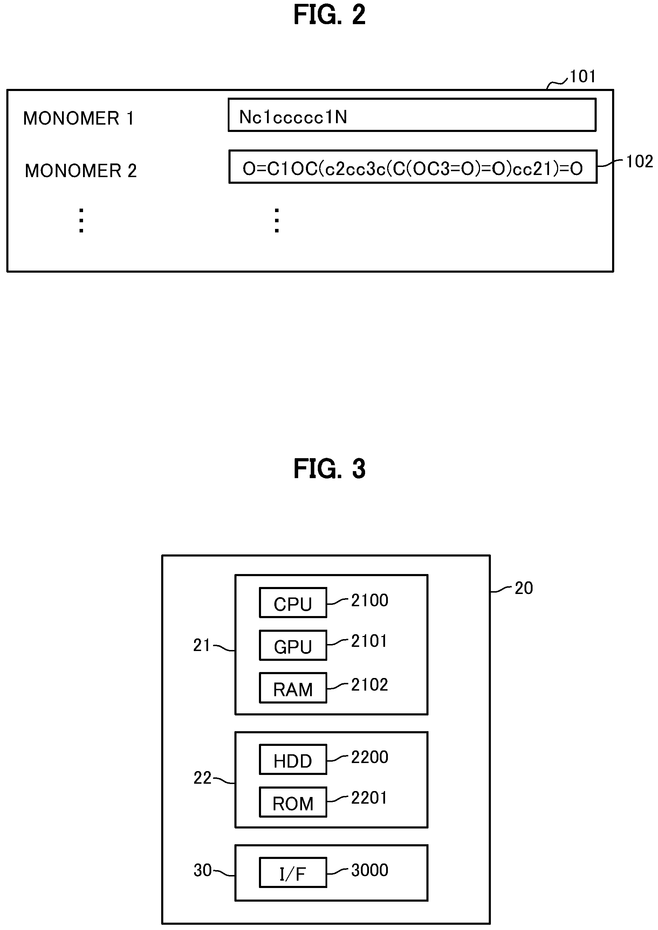

FIG. 2 is a schematic view of an input screen 101 according to one embodiment. The input screen 101 is displayed on a display device (not shown), such as a display or a monitor, for example. The input screen 101 includes input fields 102 to input input information on raw materials and the like. The molecule information described above is input into the input fields 102.

The molecule information is preferably in a format with which the three-dimensional structure of the monomer can be determined, for example. In the example shown in FIG. 2, SMILES is used for the chemical structure of each monomer, which is the molecule information. Instead of the input fields 102, files indicating the three-dimensional structures of the monomers by coordinates may be input as the molecule information. The number of pieces of molecule information to be input may be one or two or more in correspondence with the monomers that form the resin. In the example of FIG. 2, a plurality of pieces of molecule information are input (monomer 1, monomer 2, etc.).

FIG. 3 is a block diagram showing a hardware configuration of the prediction device 20 according to one embodiment. The prediction device 20 includes a computation unit 21, the storage unit 22, and the output device 30. The computation unit 21 includes a CPU 2100, a GPU 2101, a RAM 2102, and so on. The storage unit 22 includes am HDD 2200 (alternatively an SSD), a ROM 2201, and so on. The output device 30 includes an I/F 3000 and so on. The prediction device 20 is implemented by loading a predetermined program stored in the HDD 2200, the ROM 2201, or the like to the RAM 2102 and causing the CPU 2100 to execute it.

Referring back to FIG. 1, the prediction device 20 includes the computation unit 21, the storage unit 22, the reception unit 23, and the output unit 24. The reception unit 23 is connected to the input device 10 and receives molecule information on monomers being molecules that form a resin. The output unit 24 is connected to the output device 30 and outputs a predicted reaction percentage and so on to the output device 30.

For convenience of description of the computation unit 21 and the storage unit 22, a DB 222 included in the storage unit 22 will be described first, and components of the computation unit 21 will be described in the description of the storage unit 22 as appropriate. DB stands for a database, and the same applies below.

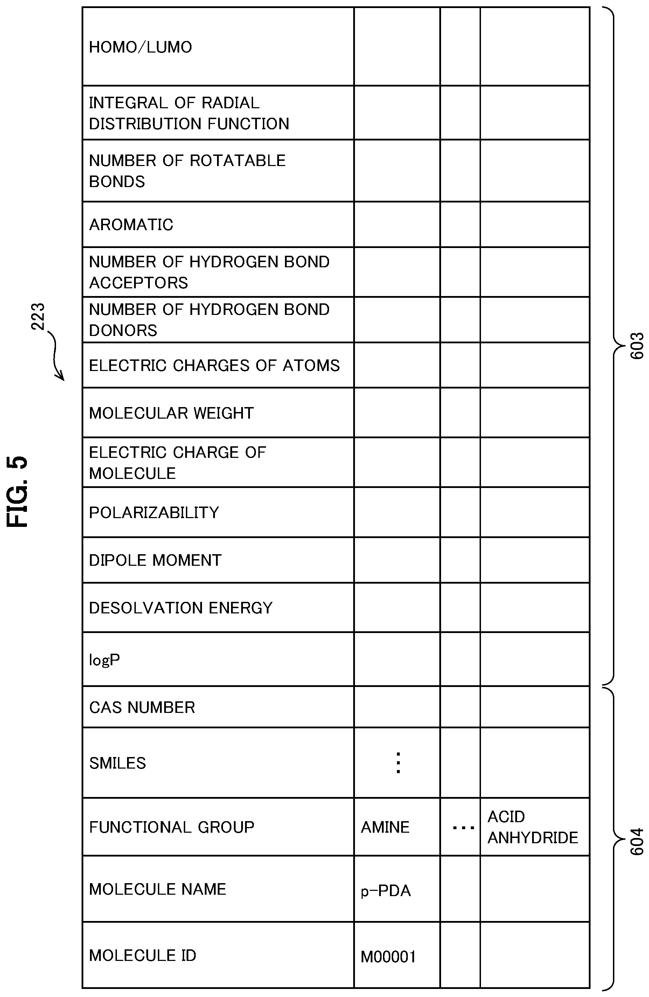

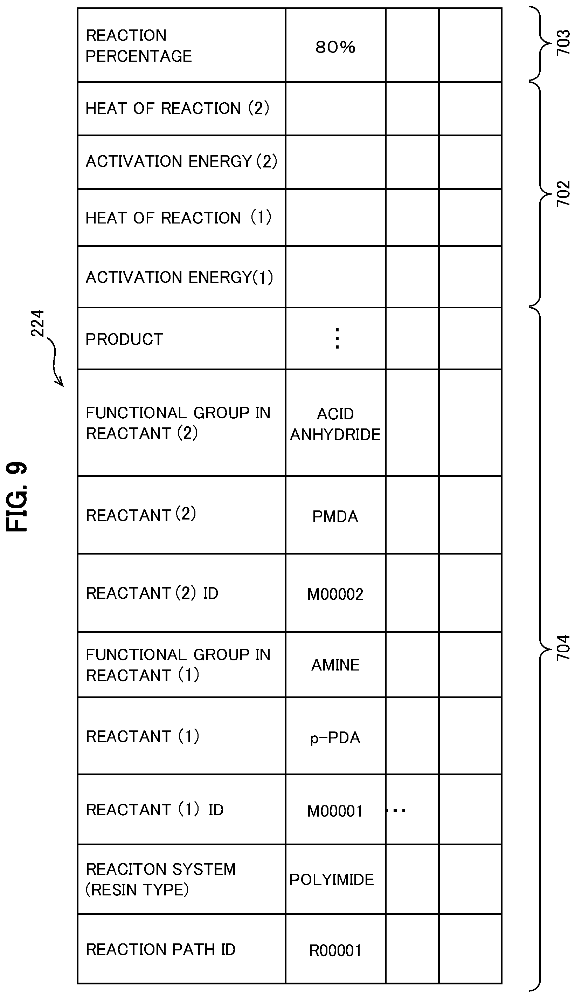

The DB 222 is a database which is included in the prediction device 20 and in which first indicators (described later), second indicators (described later), and reaction percentages are associated with one another. These are associated further with identification information of monomers corresponding to their molecule information. As will be described later in detail, in an example of the present disclosure, this identification information (e.g., a header 604 (FIG. 5)) is associated with the second indicators in a molecular property DB 223. Also, the identification information (e.g., a header 704 (FIG. 9)) is associated with the first indicators and the reaction percentage in a reaction path DB 224. In an example of the present disclosure, the DB 222 includes the molecular property DB 223, the reaction path DB 224, a prediction model DB 225, a first structure DB 226, and a second structure DB 227. Through not shown, other programs that execute calculations are stored in the storage unit 22.

The molecular property DB 223 is a database in which monomer types and molecular properties are associated with each other. The molecular properties are physical properties that affect reactivity with other reactive groups (which may be molecules such as monomers and oligomers). The molecular property DB 223 (second indicator database) includes molecular properties (second indicators) related to the frequency factor of the monomers. The frequency factor is defined by the Arrhenius equation.

While the reaction rate may be derived by differentiating the reaction percentage with respect to time, the Arrhenius equation can be used to calculate the reaction rate. The present disclosure therefore refers to the Arrhenius equation. The Arrhenius equation uses a plurality of variables, and defines the frequency factor (κ) and the activation energy (Ea). The frequency factor and the activation energy vary by the monomer or combination of monomers. The frequency factor is the frequency of collision between the monomers to be reacted with each other, and the activation energy is the energy barrier for the reaction. Features include at least indicators related to the frequency factor and the activation energy.

The molecular property DB 223 (one example of the DB 222) includes the header 604 (FIG. 5; one example of the above-described identification information) related to the types of at least one of reactive groups or resins being the products of reactions between the reactive groups. Including the header 604 makes it easier to search for the types of at least one of reactive groups or resins. Also, a monomer(s) can be obtained from the molecular property DB 223 when a prediction model to be described later is constructed. Note that FIG. 5, which does not indicate a header related to the types of resins, may include a header related to the types of both reactive groups and resins.

The header 604 includes information with which monomers, reactive groups, and the like can be classified and labeled, for example. Specific examples of the header 604 include a number as an ID representing a molecule, the name of the molecule, a functional group involved in the formation and/or dissociation of a bond(s), the SMILES representation of the molecule, the CAS number of the molecule, and so on.

In addition to the molecular property DB 223 or instead of the molecular property DB 223, at least one of the reaction path DB 224 or the prediction model DB 225 to be described later may include the header 604.

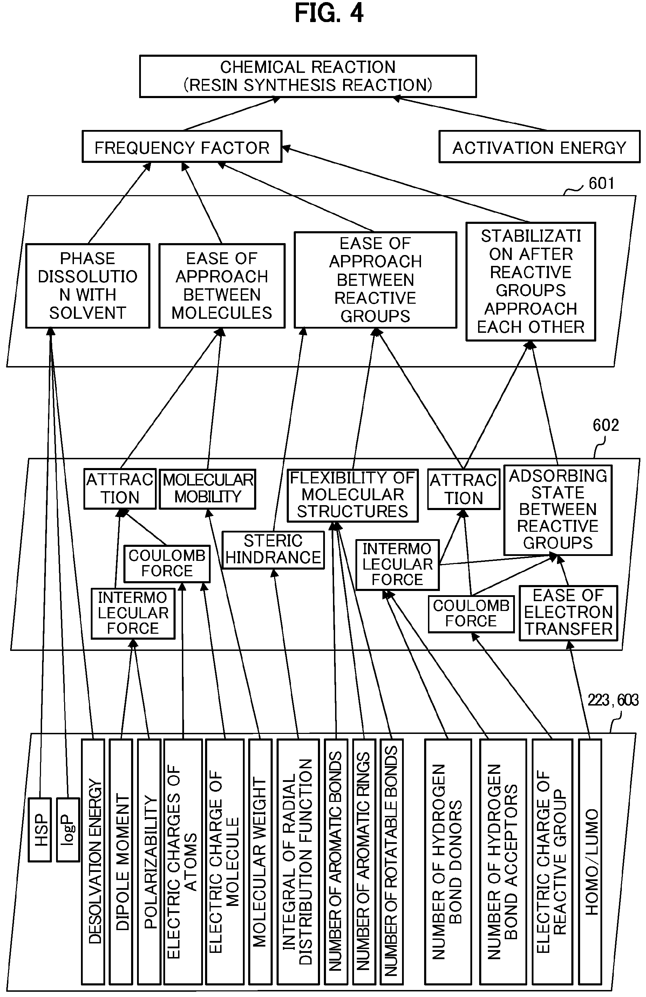

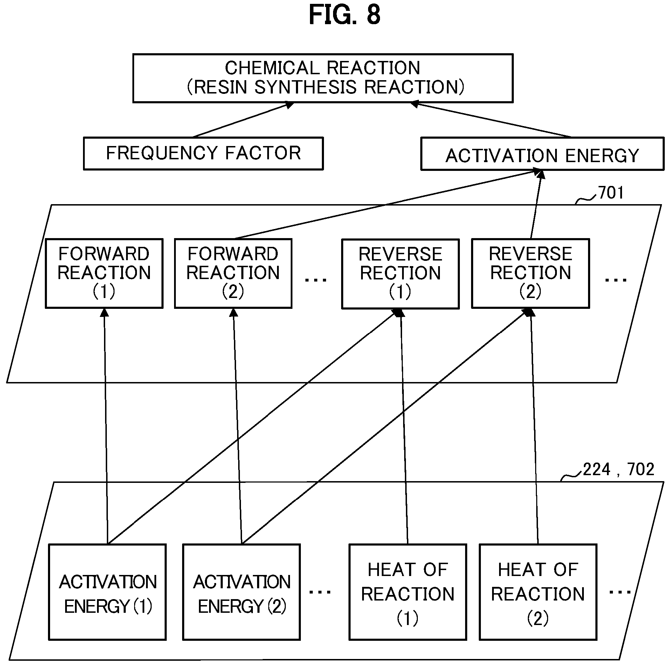

FIG. 4 is a schematic diagram showing a hierarchical structure according to one embodiment intended to extract features related to the frequency factor. As shown in the uppermost part of FIG. 4, a chemical reaction as a reaction for synthesizing a resin is governed by two indicatorsfrequency factor and activation energy. Thus, to predict the reaction percentage, it is preferable to predict it based on these two indicators. Of the two indicators, the frequency factor is described in FIG. 4 while the activation energy is described in FIG. 8 to be mentioned later.

Features related to the frequency factor are features that can reproduce phenomena up to a point when the reactive groups collide with each other and become temporarily stabilized, and are organized into the multi-layer structure shown in FIG. 4. FIG. 4 shows phenomena 601, factors 602, and features 603, for example. The phenomena 601 are phenomena that occur up to a point when the reactive groups collide with each other. The factors 602 are factors that affect the ease of occurrence of the phenomena 601. The features 603 are molecular properties as features representing the factors 602. The phenomena 601, the factors 602, and the features 603 affect one another, and these can represent the frequency factor. Thus, the features 603 are included in the molecular property DB 223 (FIG. 1) as one example of the second indicators (information on the frequency factor of the monomers).

The phenomena 601 shown in the uppermost layer are phenomena that occur up to a point when the reactive groups in the monomers collide with each other, and are organized in chronological order and listed from the left. The phenomena 601 include, for example, phenomena such as phase dissolution with a solvent, the ease of approach between the molecules (e.g., at least one of monomers or oligomers, etc.), the ease of approach between the reactive groups, and stabilization after the reactive groups approach each other. First, the monomers undergo phase dissolution with a solvent, so that the monomers are dispersed in the solution. Note that this process may not be included in the case of directing mixing the monomers. Next, the monomers or oligomers being reaction products of the monomers approach each other in a process in which the monomers or the oligomers move and get dispersed. Then, the reactive groups in the monomers or the oligomers approach and collide with each other, after which the approached state between the reactive groups is maintained until a reaction (such as formation or dissociation of a bond(s)) occurs.

Also, the uppermost layer represents a process in which the resin undergoes a structural change during the reaction, so that the reaction mode changes. For example, at the initial stage of the reaction, the monomers and the oligomers are dispersed, and the process in which these approach each other (the phenomena in the left half among the phenomena 601) is the rate-determining process. However, as the reaction progresses, the oligomers form into a mass of resin. As a result, the unreacted reactive groups approach each other owing to the thermal motions of the structures around the reactive groups (the phenomena on the right side among the phenomena 601), so that the reactive groups react with each other.

The factors 602 shown in the middle layer are factors that affect the ease of occurrence of the phenomena 601 and are organized and listed for the phenomena organized as the phenomena 601. The factors 602 include, for example, attraction and molecular mobility resulting from the intermolecular force, the Coulomb force, and the like. These are associated with the approach between the molecules among the phenomena 601. The factors 602 include attraction resulting from the steric hindrance, the flexibility of the molecular structures, the intermolecular force, the Coulomb force, and so on. These are associated with the approach between the reactive groups among the phenomena 601. The factors 602 include attraction and the adsorbing state between the reactive groups resulting from the intermolecular force, the Coulomb force, and the like, and the ease of electron transfer. These are related to the stabilization after the reactive groups approach each other among the phenomena 601. The adsorbing state mentioned here is a state where the reactive groups have approached each other. The attraction between the reactive groups and the stabilization of the electronic energy in the adsorbing state are considered to affect this stabilization. Thus, HOMO/LUMO among the features 603 to be described later, which is related to the ease of electron donation and acceptance, is used as a molecular property that represents the stabilization of the electronic energy.

The features 603 shown in the lowermost layer are the second indicators related to the frequency factor and are features that can represent the frequency factor. The features 603 are molecular properties that affect the reaction between the molecules during the reaction between the reactive groups included in the molecules.

The features 603 include, for example, HSP (Hansen solubility parameters), logP, and the desolvation energy. These are related to the phase dissolution with a solvent among the phenomena 601. The features 603 include, for example, the dipole moment and the polarizability, which affect the attraction term. These are related to the intermolecular force forming the attraction related to the ease of approach between the molecules among the phenomena 601. The features 603 include, for example, the electric charges of the atoms and the electric charges of the molecules. These are related to the attraction related to the ease of approach between the molecules among the phenomena 601. The features 603 include, for example, the molecular weights, which affect the mobility. These are related to the molecular mobility related to the ease of approach between the molecules among the phenomena 601.

The features 603 includes, for example, an integral of the radial distribution function. This is related to the steric hindrance around the reactive groups among the factors 602. The steric hindrance is defined as a reduction in reactivity as a result of another atom in a molecule approaching the periphery of its reactive group. Hence, the steric hindrance can be represented by the radial distribution function of the periphery of the atom at the reaction center. The radial distribution function is a function representing the distribution of atoms located around an origin such as any atom included in a reactive group or the center of gravity of the reactive group (existence probability) over a distance r from that origin. While the function may be used as is as a feature, the integral (area) over the distance from the center atom may be used for simplicity.

The features 603 include, for example, the number of aromatic bonds, the number of aromatic rings, and the number of rotatable bonds. These are related to the flexibility of the molecular structures (the mobility of the reactive groups) among the factors 602. The features 603 include the number of hydrogen bond donors and the number of hydrogen bond acceptors as attraction terms for the reactive groups. These are related to the intermolecular force (such as hydrogen bonding and the Coulomb force) related to the attraction related to the approach between the reactive groups and the stabilization after the reactive groups approach each other among the phenomena 601. The features 603 include the electric charges of the reactive groups as attraction terms for the reactive groups. These are related to the Coulomb force related to the approach between the reactive groups and the stabilization after the reactive groups approach each other among the phenomena 601. The features 603 include HOMO/LUMO or the like, for example. This is related to the ease of electron transfer among the factors 602.

As described above, the phenomena 601, the factors 602, and the features 603 are related to one another. Thus, for example, the approach between the molecules among the phenomena 601 can be represented by the attraction (such as the intermolecular force and the Coulomb force) and the molecular mobility among the factors 602, as described above. Further, the attraction and the molecular mobility among the factors 602 can be represented by the dipole moments, the polarizability, and the electric charges of the molecules among the features 603. This applies similarly also to the other phenomena 601, factors 602, and features 603 in FIG. 4.

In practical applications, it is possible to use other features than those shown in FIG. 4 that match the reaction mechanism. However, it is important to systematically organize the phenomena through hierarchical structuring. The prediction models can be expected to more accurate by changing the reaction mechanism to a more appropriate one based on the reaction system and performing at least one of addition or deletion of a feature(s) 603 based on that.

FIG. 5 is a schematic diagram of the molecular property DB 223 according to one embodiment. The molecular property DB 223 includes features 603 determined based on the reasons described above with reference to FIG. 4. The structure of the molecular property DB 223 is not limited to a table form as shown in FIG. 5, and may be a graph form that includes nodes representing coefficients of correlation between the features 603, causal relationships, and data generation procedures, or the like. The molecular property DB 223 further includes the above-described header 604.

As described above, the molecular property DB 223 includes the second indicators related to the frequency factor. The second indicators include at least one of the structure of the monomer corresponding to its molecule information or molecular properties (e.g., the above-described features 603) determined from the electronic state of the monomer corresponding to the molecule information. The molecular properties here are physical properties that affect the reactivity with other monomers. FIG. 5 includes both of these as an example. Including at least one of these makes it possible to determine the frequency factor corresponding to the monomer. The example shown in FIG. 5 includes the structures of monomers in SMILES, and includes the molecular properties determined from the electronic states of the monomers as HOMO/LUMO.

The second indicators include at least one of: at least one of an attraction or a motion exerted on at least one of monomers or reactive groups; or the steric hindrance of the reactive group against another monomer. A reactive group is a group that is included in a monomer and reacts with another monomer. Including at least one of these makes it possible to determine the frequency factor corresponding to the monomer. In the example shown in FIG. 5, the attraction exerted on monomers is, for example, the dipole moment, the polarizability, the electric charge of the molecule, and the electric charges of the atoms. The motion exerted on monomers (molecular mobility) is, for example, the molecular weight. The attraction exerted on reactive groups is the number of hydrogen bond acceptors and the number of hydrogen bond donors. The motion exerted on reactive groups is, for example, the number of rotatable bonds.

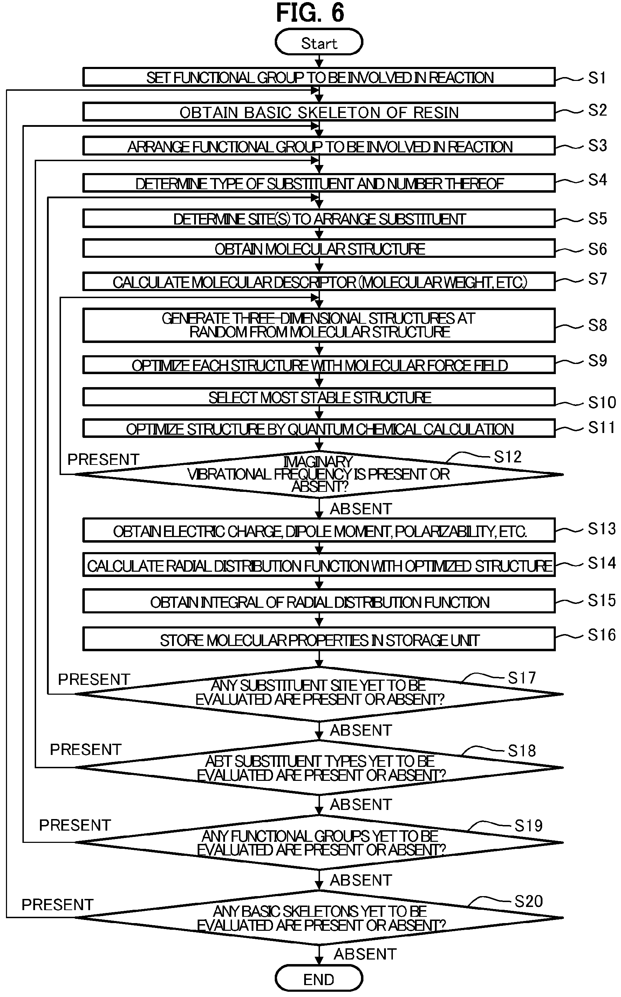

FIG. 6 is a flowchart showing a method of constructing the molecular property DB 223 according to one embodiment. A second construction unit 216 (FIG. 1) can construct the molecular property DB 223 by using a program that can be used for molecular simulation, generation of molecular descriptors, and the like, for example. For example, various software can be used depending on the types of features, such as RDKit, which is a Python library, Gaussian and GAMESS, each of which is quantum chemical calculation software; and LAMMPS, which is a molecular dynamics simulation software. Note that the second construction unit 216 may not be included, and a molecular property DB 223 constructed in advance may be used.

As the phenomena 601 in FIG. 4 described above show, for example, the second construction unit 216 organizes the phenomena 601 which occurs during a reaction between reactive groups in chronological order. Further, as the factors 602 in FIG. 4 described above show, for example, the second construction unit 216 identifies the factors 602 that affect the phenomena 601. By doing so, the second construction unit 216 constructs the molecular property DB 223 (one example of a second indicator DB) including the features 603 as molecular properties representing the factors 602 (one example of the second indicators). In this way, it is possible to systematically figure out the chemical reactions of resins and determine the second indicators. A specific method of constructing the molecular property DB 223 will now be described below with reference to FIG. 6.

Step S1: First, the second construction unit 216 sets a functional group (reactive group) to be involved in a reaction. The items to be set are the type of functional group and its arrangement. The type of functional group is determined by calling it from a functional group DB (not shown) stored in the storage unit 22 (FIG. 1). The type of functional group is any functional group to be involved in formation of a resin, such as an amino group, an acid anhydride, or the isocyanate group, for example. Also, the number of functional groups, which may be any number, is preferably one or more and more preferably two or three. Also, a plurality of types of functional groups may be selected. The arrangement of the functional group is its binding site in the basic skeleton in step S2 to be described later.

Step S2: The second construction unit 216 obtains the basic skeleton of a resin formed of monomers from a basic skeleton DB (not shown) stored in the storage unit 22. The basic skeleton of the molecule is, for example, a benzene ring, an alkyl group, or the like. The number of benzene rings, the number of carbon atoms in the alkyl group, the bond order, and the like may be any numbers and order.

Step S3: Based on the type of functional group and its arrangement determined in step S1, the second construction unit 216 arranges the functional group of the determined type at the determined site in the basic skeleton of the resin determined in step S2. The arrangement is done by simulation. As a result, the reactive functional group (reactive group) forming the resin can be arranged in the basic skeleton of the resin. Thus, the molecular structure of the resin is obtained.

In order to get every arranged functional group involved in the function, the site to arrange the functional group is preferably processed as follows. First of all, the functional group forming the resin is arranged at a terminal of the molecular structure as a rule. However, the functional group can be inserted within the basic skeleton as along as the functional group can be involved in the reaction even without being arranged at a terminal of the molecule. Examples of such a functional group include an amino group and the like. Since the secondary amino group and the tertiary amino group may be reactive, an amino group may be inserted within the basic skeleton. Also, in order to prevent the reactive group from losing its reactivity, it is preferable to keep the functional group from binding to another or other ones in the same molecule.

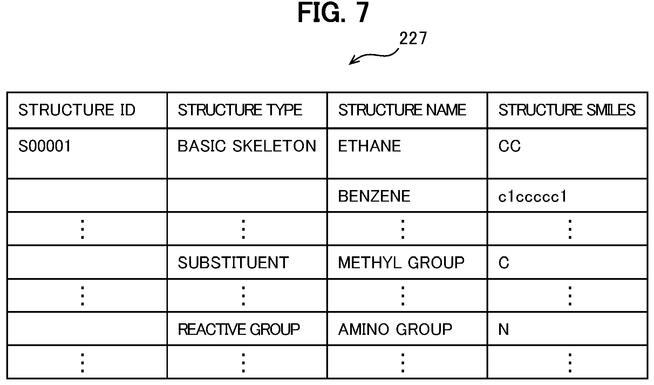

Step S4: The second construction unit 216 determines a type of substituent and the number thereof based on the second structure DB 227 (FIG. 7) for substituents stored in the storage unit 22 (FIG. 3). In the present disclosure, a substituent means a molecular structure that is not involved in a resin formation reaction. As the substituent, it is possible to use any substituent commonly included in organic compounds, such as a methyl group, an ethyl group, an ether, or a hydroxy group, for example. A plurality of types of substituents can be used. Note that the substituent is different in type from the functional group to be involved in the reaction. Also, as a rule, the substituent does not bind to the functional group. This is to prevent the functional group from losing its reactivity or turning into another functional group as a result of the functional group and the substituent binding to each other.

FIG. 7 is a schematic diagram of the second structure DB 227 according to one embodiment. The second structure DB 227 is a DB storing information on the names, structures, and the like of the skeletons of molecules and their functional groups and substituents, and of resins to be produced. The reactive group, the basic skeleton of the resin, and the substituent used in steps S1 to S4 in FIG. 6 are obtained from the second structure DB 227. The second structure DB 227 includes IDs being numbers assigned to pieces of data, structure types (basic skeleton, substituent, etc.), structure names, notations from which three-dimensional structures can be generated (written in SMILES in FIG. 7), and the like. Note that a separate DB may be created for each structure type.

Step S5: Referring back to FIG. 6, based on the type of substituent and the number thereof determined in step S4, the second construction unit 216 determines the site(s) to arrange the substituent.

Step S6: Based on the results of steps S1 to S5, the second construction unit 216 obtains a molecular structure. Any format can be used to obtain the molecular structure as long as the format can represent the molecular structure in two or more dimensions. Examples include a file describing the SMILES representation or the molecules coordinates, and the like.

Step S7: Based on the obtained molecular structure, the second construction unit 216 calculates the molecular descriptor of the substituent. As a result, two-dimensional molecular properties are derived. The molecular properties derived in step S7 are, for example, the molecular weight, the number of hydrogen bond donors, the number of hydrogen bond acceptors, logP, the electric charge of the molecule, aromatic, the number of rotatable bonds, and so on.

Step S8: Based on the molecular structure obtained in step S6, the second construction unit 216 generates a plurality of three-dimensional structures of the resin at random.

Step S9: For each of the molecular structures generated in step S8, the second construction unit 216 optimizes the structure with a molecular force field. At this time, the second construction unit 216 obtains an energy value of each molecular structure. The structural optimization can be executed via molecular simulation, such as molecular mechanics calculation.

Step S10: The second construction unit 216 compares the energy values of the molecular structures obtained in step S9 with each another and selects the structure with the lowest energy as the most stable structure.

Step S11: The second construction unit 216 further performs structural optimization on the most stable structure obtained in step S10 by using quantum chemical calculation.

Step S12: The second construction unit 216 obtains the vibrational frequency of the structure subjected to the structural optimization. If there are only positive vibrational frequencies, the structure is the most stable structure among the candidate structures for the molecule. The second construction unit 216 therefore selects the structure as the most stable structure, and the second construction unit 216 executes the next step S13. On the other hand, if an imaginary vibrational frequency is present, the structures is not the true most stable structure. The second construction unit 216 therefore returns to step S8 and searches for the most stable structure again.

Step S13: The second construction unit 216 obtains molecular property values of the most stable structure obtained in step S11, such as the electric charge of the reactive group, the dipole moment, and the polarizability, which can be derived by quantum chemical calculation.

Step S14: The second construction unit 216 calculates the radial distribution function of the periphery of the reactive group in the most stable structure obtained in step S11. The radial distribution function is calculated as described above with reference to FIG. 4.

Step S15: The second construction unit 216 integrates the radial distribution function obtained in step S14 to obtain an integral.

Step S16: The second construction unit 216 stores all of the data obtained in the above calculations in the molecular property DB 223 (FIG. 1) in the storage unit 22 (FIG. 1). In doing so, the second construction unit 216 also newly assigns IDs and the like. The data to be stored include, for example, the molecular properties (features 603 (FIG. 4) but may additionally include the frequency factor and the like.

Step S17: The second construction unit 216 determines whether there are any substituent sites yet to be evaluated. The second construction unit 216 iterates steps from step S5 if there is a site yet to be evaluated, and proceeds to step S18 if there is none.

Step S18: The second construction unit 216 determines whether there are any substituent types yet to be evaluated. The second construction unit 216 iterates steps from step S4 if there is a type yet to be evaluated, and proceeds to step S19 if there is none.

Step S19: The second construction unit 216 determines whether there are any functional groups yet to be evaluated. The second construction unit 216 iterates steps from step S3 if there is a functional group to be evaluated, and proceeds to step S20 if there is none.

Step S20: The second construction unit 216 determines whether there are any basic skeleton yet to be evaluated. The second construction unit 216 iterates steps from step S2 if there is a basic skeleton yet to be evaluated, and terminates the process flow if there is none.

In the example shown in FIG. 6, molecular mechanics calculation is executed in step S9, and quantum chemical calculation is executed in step S11. However, the calculations for performing the molecular simulation are not limited to these examples. Specifically, the second construction unit 216 can construct the molecular property DB 223 from molecule information based on at least one of descriptor calculation, quantum chemical calculation, molecular mechanics calculation, molecular dynamics calculation, first-principles calculation, or first-principles molecular dynamics calculation based on the structure of the monomer. The molecular property DB 223 can be constructed by at least one of these calculations.

Also, the second construction unit 216 can construct the molecular property DB 223 through at least one of calculation of the molecular descriptor or execution of molecular simulation for the molecular structure of the resin obtained in step S6. The molecular structure here is the molecular structure of the resin obtained in step S6 by arranging the reactive functional group forming the resin in the basic skeleton of the resin. The molecular property DB 223 can be constructed by at least one of these calculations. In an example of the present disclosure, the molecular descriptor is calculated in step S7, and the molecular simulation is executed in step S9.

Referring back to FIG. 1, the reaction path DB 224 is a DB including the first indicators and a predetermined reaction percentage 703 for each of a plurality of combinations of monomers (FIG. 9). The first indicators are indicators related to the activation energy of the monomers. The activation energy is defined by the Arrhenius equation. The reaction percentage includes at least one of a measured value or a calculated value. The calculated value can be calculated by molecular simulation, such as quantum chemical calculation, for example. As will be described in detail later, a prediction unit 214 performs reaction percentage prediction by using a reaction percentage included in the reaction path DB 224.

FIG. 8 is a schematic diagram showing a hierarchical structure according to one embodiment intended to extract features related to the activation energy. Chemical reactions are governed also by the activation energy, as with the frequency factor described above. Thus, as with the frequency factor, phenomena are organized for the activation energy through hierarchical structuring, as shown in FIG. 8. In this way, features can be extracted. Phenomena 701 are phenomena that occur during a reaction between monomers, and features 702 are features related to the ease of reaction. The phenomena 701 and the features 702 affect each other, and these can represent the activation energy. Thus, the features 702 are included in the reaction path DB 224 (FIG. 1) as the first indicators (information on the activation energies of monomers).

In chemical reactions, a forward reaction and a reverse reaction progress simultaneously. Also, chemical reactions may progress through a plurality of steps, e.g., via an intermediate. Thus, the phenomena 701 include a forward reaction (1), a forward reaction (2), a reverse reaction (1), and a reverse reaction (2), for example. In this example, the forward reaction (1) and the reverse reaction (1) progress simultaneously to produce an intermediate from the monomers and, starting from the intermediate, the forward reaction (2) and the reverse reaction (2) progress simultaneously to form a resin from the intermediate.

Activation energy affects the ease of occurrence of forward reactions, and activation energy, heat of reaction, and so on affect the ease of occurrence of reverse reactions. For this reason, the features 702 include an activation energy (1) that affects the forward reaction (1) and the reverse reaction (1), and an activation energy (2) that affects the forward reaction (2) and the reverse reaction (2). The features 702 further include heat of reaction (1) that affects the reverse reaction (1) and heat of reaction (2) that affects the reverse reaction (2). Note that the features related to activation energy (e.g., the activation energy (1) and the activation energy (2)) may be calculated values obtained by using molecular simulation, such as quantum chemical calculation or first-principles calculation, or literature values.

Note that, in an example of the present disclosure, an activation energy is used as a feature for each reaction, while the present disclosure is not limited to this. For example, when the activation energy (1) and the activation energy (2) are included, it is preferable to treat the activation energy (1) and the activation energy (2) as independent features. This makes it easier to represent the chemical reaction with activation energies.

Also, in another embodiment, only the highest activation energy among the activation energies for the respective sets of reactions can be used as a feature, while the present disclosure is not limited to this case. This is because the reaction with the highest activation energy is the rate-determining step. Thus, the number of features related to activation energy is one even when the reaction involves a plurality of steps. This can reduce the load of learning.

FIG. 9 is a schematic diagram of the reaction path DB 224 according to one embodiment. The reaction path DB 224 is in a table form in the example of FIG. 9 but may be in a graph form. The reaction path DB 224 includes the above-described features 702 as the first indicators. The features 702 (first indicators) include at least one of the activation energies of monomers or the heats of reaction in the formation of a resin from the monomers. These affect the chemical reaction in the formation of the resin from the monomers, as described above. Thus, including at least one of these makes it possible to construct the reaction path DB 224.

The reaction path DB 224 includes the reaction percentages 703 for systems whose reaction percentages are known. The reaction percentages 703 are used as training data in the creation of the prediction models (described later) .

The reaction path DB 224 includes the header 704 (one example of the above-described identification information), like the above-described header 604 (FIG. 5). The header 704 is related to the types of at least one of reactive groups or a resin as the product of a reaction between the reactive groups. Data can be obtained from the reaction path DB 224 during the creation of the prediction models to be described later. FIG. 9 exemplarily shows an amine and an acid anhydride as substances related to reactive groups, p-PDA as a reactant (1) being a monomer, and PMDA as a reactant (2) being a monomer, as an example. Including the header 704 makes it easier to extract at least one of target reactive groups or a target resin.

Further, the header 704 includes at least one of the classification of the combination of the plurality of monomers, or information with which the combination can be labeled. The header 704 includes information on molecules such as numbers as IDs representing the molecules, the names of the molecules, functional groups involved in the formation or dissociation of a bond(s), the SMILES representations of the molecules, and the CAS numbers of the molecules and, in addition, the resin type (e.g., polyimide).

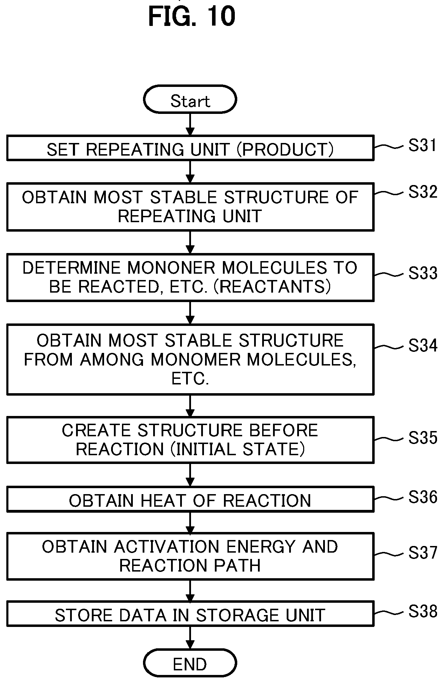

FIG. 10 is a flowchart showing a method of constructing the reaction path DB 224 according to one embodiment. The flowchart shown in FIG. 10 is a flowchart showing a method of deriving the first indicators (the activation energies, etc.) for a resin whose reaction percentage has already been obtained, as training data for the later-described prediction models. A first construction unit 215 (FIG. 1), for example, can construct the reaction path DB 224. Note that the first construction unit 215 may not be included, and a reaction path DB 224 constructed in advance may be used.

The first construction unit 215 constructs the reaction path DB 224 (FIG. 1; one example of a first indicator database) by organizing the phenomena 701 (FIG. 8) which occur during a reaction between reactive groups in chronological order, and evaluating the ease of occurrence of the phenomena 701. In this way, it is possible to systematically figure out the formation reactions of resins and determine the first indicators. A specific method of constructing the reaction path DB 224 will now be described below with reference to FIG. 10.

Step S31: The first construction unit 215 sets a product for a resin with monomers whose reaction percentage is known. The product refers to a repeating unit being a structure formed by a reaction between monomers each being a single molecule.

Step S32: The first construction unit 215 generates three-dimensional structures based on information on the repeating unit obtained in step S31, and then obtains the most stable structure. The method of obtaining the most stable structure is similar to the method described in step S10, etc. for the above-described molecular property DB 223. The most stable structure obtained here is the final state of the resin formed as a result of the monomers reacting with each other.

Step S33: The first construction unit 215 determines reactants based on information on the repeating unit obtained in step S31 (the type of the resin including the repeating unit, etc.). The reactants are molecules being monomers that form the repeating unit, and their molecular structures. From the type of the resin including the repeating unit, functional groups (reactive groups) to be involved in the reaction are determined, and the reactants are determined based on them.

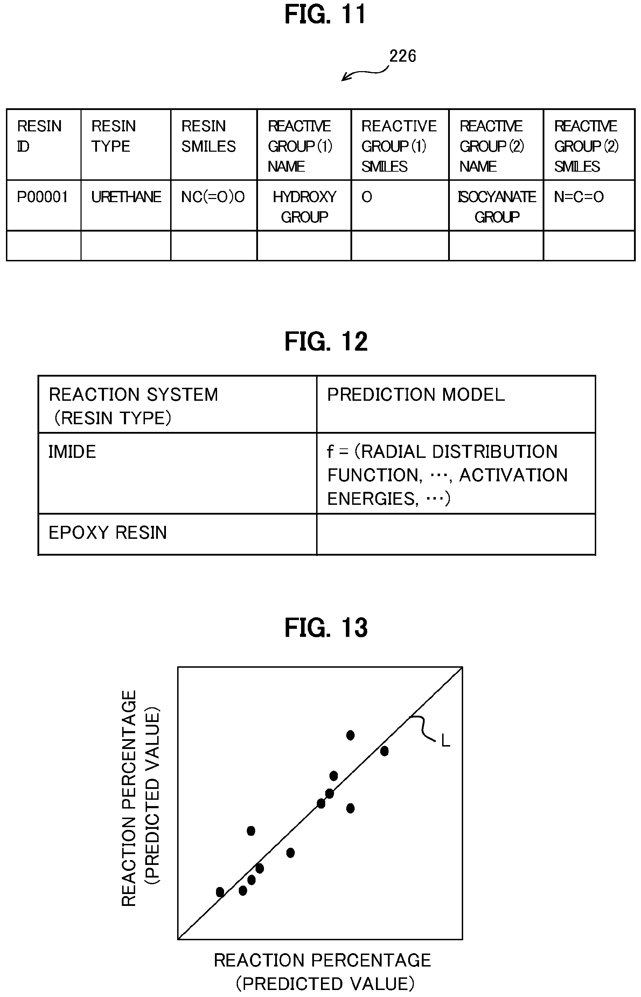

FIG. 11 is a schematic diagram of the first structure DB 226 according to one embodiment. The first structure DB 226 is a DB storing information on the names, structures, and the like of the skeletons of molecules and their functional groups and substituents, and of resins to be produced. In step S33 (FIG. 10), the functional groups to be involved in the reaction are called from the first structure DB 226. The first structure DB 226 includes IDs being numbers assigned to pieces of data, notations from which three-dimensional structures of structures can be generated (written in SMILES in FIG. 11), the names and SMILES of reactive groups in multi-step reactions, and so on. Note that a separate DB may be created for each structure type.

Step S34: Referring back to FIG. 10, the first construction unit 215 generates three-dimensional structures based on information on the monomer molecules and the like (reactants) obtained in step S33, and then obtains the most stable structure. The method of obtaining the most stable structure is similar to the method described in step S10, etc. for the above-described molecular property DB 223.

Step S35: From the most stable structure obtained in step S34, the first construction unit 215 generates a plurality of structures in which the functional groups to be involved in the reaction (reactive groups) are arranged close to each other, and performs structural optimization on all of the generated structures by using molecular simulation, such as molecular mechanics calculation. For each structure, energy is derived, and the structure with the lowest energy is selected. As a result, a structure before the reaction (initial state, initial structure) is created. The first construction unit 215 further performs structural optimization on the created structure by quantum chemical calculation to obtain an energy value.

As described above, the first construction unit 215 determines a three-dimensional structure of a resin whose reaction percentage is known, and the monomers forming the resin. Then, the first construction unit 215 generates a plurality of three-dimensional structures in which the reactive groups in the monomers to be subjected to reaction percentage prediction are arranged close to each other, followed by structural optimization, to thereby construct the reaction path DB 224. In this way, structures before the reaction can be created.

Step S36: The first construction unit 215 calculates the difference between the energy obtained in step S35 (the energy of the structure in the initial state) and the energy obtained in step S31 (the energy of the structure in the final state) to obtain the heat of reaction.

Step S37: The first construction unit 215 obtains the activation energy and the reaction path based on the structure in the initial state obtained in step S35 and the structure in the final state obtained in step S31. The obtaining can be done by a reaction path determination unit 212 (FIG. 1). The reaction path determination unit 212 determines the structure of the resin from its monomers, and determines the reaction path from the monomers to the resin by molecular simulation. By determining the reaction path with the reaction path determination unit 212, the activation energy and the heat of reaction can be determined.

A specific determination method will now be described. The reaction path determination unit 212 determines the functional groups to be involved in the reaction (reactive groups) from molecule information. The reaction path determination unit 212 determines the resin type (e.g., polyimide) based on the combination of functional groups, thereby determining the molecular structure of the resin. Then, by the above-described method, the reaction path determination unit 212 determines the structure in the initial state based on the molecule information and determines the structure in the final state based on the molecular structure of the resin. The reaction path determination unit 212 can calculate the activation energy and the heat of reaction from the structures in the initial state and the final state by performing a reaction path search using molecular simulation, such as quantum chemical calculation or first-principles calculation, for example. As the reaction path search method, the GRRM (Global Reaction Route Mapping) method, the NEB (Nudged Elastic Band) method, the String method, a method created by developing any of these methods, or the like can be used, for example.

Step S38: The first construction unit 215 stores all of the data obtained in the above in the reaction path DB 224 (FIG. 1) in the storage unit 22 (FIG. 1). In doing so, the first construction unit 215 also newly assigns IDs and the like. The stored data includes the activation energy (1), the activation energy (2), the heat of reaction (1), the heat of reaction (2) (the features 702 (FIG. 8)), and the like, for example.

Incidentally, when the reaction is a multi-step reaction, the first construction unit 215 iterates steps S33 to S38 as many times as the number of reactions. If the reaction involves two steps and the reaction progresses such that an intermediate is produced from reactants (monomers) and a product is produced from the intermediate, the first construction unit 215 performs the processes with the reactants as the initial state and the intermediate as the final state in the first reaction. Also, the first construction unit 215 performs the processes with the intermediate as the initial state and the product as the final state in the second reaction.

As described above, in the case where a reaction to form a resin from monomers progresses through a plurality of steps of reactions via an intermediate, the first construction unit 215 constructs the reaction path DB 224 through a plurality of separate steps from the molecules to the intermediate and from the intermediate to the resin. This can prevent complication of phenomena due to a plurality of reactions being associated with each other, and thus simplify the calculation.

Referring back to FIG. 1, the prediction model DB 225 (one example of the DB 222) is a DB including prediction models that predict reaction percentages. By using a prediction model, the reaction percentage of monomers that form a resin can be predicted from molecule information input by a user. The prediction model DB 225 further includes resin types. Examples of the resin types include polyimide and epoxy resin.

FIG. 12 is a schematic diagram of the prediction model DB 225 according to one embodiment. In the prediction model DB 225, a prediction model is included for each individual resin type (reaction system) in the DB 222. In the example shown in FIG. 12, in the case of producing, for example, an imide resin, the prediction model is a function including the second indicators such as the radial distribution function and the first indicators such as the activation energies as variables.

A prediction model construction unit 213 (FIG. 1) constructs the prediction models. The prediction model construction unit 213 constructs the prediction models by machine learning in which the above-described first indicators and second indicators are features and the above-described reaction percentage is a response variable. Incidentally, of the features used in the machine learning, the features related to the first indicators are the above-described features 702 (FIG. 8), and the features related to the second indicators are the above-described features 603 (FIG. 4). Thus, in the prediction model construction using the machine learning, the reaction percentage can be predicted by inputting the first indicators and the second indicators into the prediction model. The first indicators are included in the reaction path DB 224 (FIG. 1), for example, and include the activation energies and the heats of reaction, for example. The second indicators are included in the molecular property DB 223, for example, and include molecular properties related to the frequency factor. The machine learning can use any algorithm. The machine learning is preferably supervised learning, but may be unsupervised learning or reinforcement learning.

It is preferable that the prediction model construction unit 213 construct a different prediction model for each resin. Since each resin's reaction is different, doing so allows for construction of more accurate prediction models.

The reaction percentages used in the machine learning include, for example, measured values obtained by actually reacting the monomers with each other. Including the measured values enables a predicted reaction percentage to be closer to the actual reaction percentage and allows for an improvement in prediction accuracy.

The reaction percentages used in the machine learning include, for example, calculated values obtained by molecular simulation assuming that the monomers are reacted with each other. The accuracy of the calculated values obtained by the molecular simulation is high to a certain extent. Thus, a large number of calculated values can be prepared by the molecular simulation, and the large number of calculated values can be used to construct a prediction model. This allows for an improvement in the prediction accuracy of the prediction model.

A molecular property determination unit 211 (FIG. 1) can obtain the molecular properties to be used in the machine learning (second indicators). The molecular property determination unit 211 calculates the molecular properties to be used in the machine learning based on molecule information which is identification information identifying monomer molecules (molecule names, rational formulae, molecule coordinate data, etc.). The calculation can be executed by molecular simulation, such as descriptor calculation, quantum chemical calculation, or molecular dynamics calculation, for example. The molecular simulation can be performed by executing any program.

Also, the reaction path determination unit 212 (FIG. 1) can obtain the activation energies (first indicators) and the reaction percentages to be used in the machine learning.

FIG. 13 is a graph comparing predicted values and measured values when prediction models are created in one embodiment. In FIG. 13, the horizontal axis (x axis) represents the reaction percentage (predicted value), the vertical axis (y axis) represents the reaction percentage (measured value), and a straight line L represents a graph indicating y = x. The graph of FIG. 13 is a diagram plotting reaction percentages obtained from resins actually produced with monomers in input molecule information (measured values) and reaction percentages predicted by using the molecule information and prediction models (predicted values). As shown in FIG. 13, each of the plots is present around the graph of y = x, which is the straight line L. Thus, the predicted values have values close to the measured values, indicating that the prediction accuracy of the prediction models is high.

Referring back to FIG. 1, the computation unit 21 includes the molecular property determination unit 211, the reaction path determination unit 212, the prediction model construction unit 213, the prediction unit 214, the first construction unit 215, and the second construction unit 216. Of these, the molecular property determination unit 211, the reaction path determination unit 212, the prediction model construction unit 213, the first construction unit 215, and the second construction unit 216 have been described with reference to FIG. 12, etc. mentioned above, and description thereof will therefore be omitted.

From the first indicators and the second indicators of monomers determined from the molecule information on the monomers and the DB 222, the prediction unit 214 predicts the reaction percentage. The first indicators are indicators related to the activation energies, as described above, and are determined by the reaction path determination unit 212. The reaction path determination unit 212 (first determination unit) determines the first indicators to be used by the prediction unit 214 by obtaining information corresponding to molecule information received by the reception unit 23 from the DB 222 (in particular, the reaction path DB 224). By including the reaction path determination unit 212, information to be used in the reaction percentage prediction can be obtained from the input molecule information.

The reaction path determination unit 212 (first determination unit) determines the structure of a resin from the monomers in the molecule information, and determines the reaction path from the monomers to the resin by molecular simulation. In this way, the reaction path can be determined.

The second indicators are molecular properties related to the frequency factor, as described above, and are determined by the molecular property determination unit 211. The molecular property determination unit 211 (second determination unit) determines the second indicators to be used by the prediction unit 214 by obtaining information corresponding to the molecule information received by the reception unit 23 from the DB 222 (in particular, the molecular property DB 223). By including the molecular property determination unit 211, information to be used in the reaction percentage prediction can be obtained from the input molecule information. The prediction unit 214 inserts the obtained first indicators and second indicators into a prediction model in the prediction model DB 225 to predict the reaction percentage.

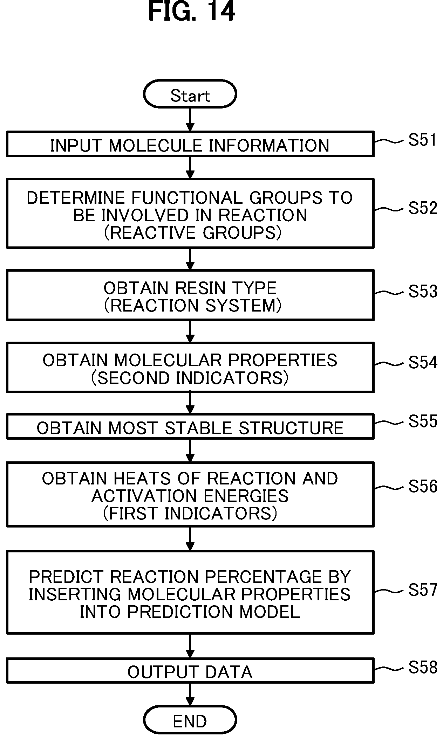

FIG. 14 is a flowchart showing a prediction method of the present disclosure. The prediction device 20 (FIG. 1) can execute the flow shown in FIG. 14. Thus, FIG. 14 will be described with reference to FIG. 1 etc., as appropriate. The prediction method of the present disclosure and a reaction percentage predicted by the prediction device 20 are intended to predict the reaction percentage of monomers whose reaction percentage is not known for a reaction system for which the above-described prediction model has already been constructed.

Step S51: The user inputs molecule information on monomers serving as raw materials with the input device 10.

Step S52: The molecular property determination unit 211 and the reaction path determination unit 212 determine functional groups to be involved in the reaction (reactive groups) based on the molecule information (the names, rational formulae, structure information, etc. of the molecules to be reacted).

Step S53: The molecular property determination unit 211 and the reaction path determination unit 212 determine the resin type (reaction system) based on the determined reactive groups. In doing so, the molecular property determination unit 211 and the reaction path determination unit 212 call the resin type from the first structure DB 226 (FIG. 11) and the second structure DB 227 (FIG. 7) as appropriate.

Step S54: The molecular property determination unit 211 determines molecular properties as the second indicators based on the molecule information. The determination can be done by executing, for example, steps from step S5 in the flowchart described with reference to FIG. 6 mentioned above.

Step S55: The reaction path determination unit 212 obtains the product resin's most stable structure based on the structures of the monomers in the molecule information input in step S51 described above and the resin type obtained in step S53 described above. The most stable structure can be obtained in a similar manner to the method described with reference to FIGs. 6 and 10 mentioned above. The product resin's structure is a structure after the reaction between the monomers, each of which is a single molecule (two molecules if there is only one type of monomer), as in the construction of the reaction path DB 224.

Step S56: The reaction path determination unit 212 obtains the heats of reaction and the activation energies as the first indicators based on the molecular properties and the most stable structure obtained in steps S54 and S55. The obtaining can be done by executing, for example, steps from step S5 in the flowchart described with reference to FIG. 10 mentioned above.

Step S57: The prediction unit 214 inserts the molecular properties, the heats of reaction, and the activation energies determined in steps S54 and S56 into the prediction model corresponding to the resin type determined in step S53 to predict the reaction percentage. Step S57 is a prediction step of predicting the reaction percentage from the first indicators and the second indicators of the monomers that form the resin determined based on the molecule information on the monomers received in step S51 and from the DB 222. Incidentally, the DB 222 is a DB in which the above-described first indicators of monomers as molecules that form resins, the above-described second indicators of the monomers as the molecules that form the resins, and the above-described reaction percentages are associated with one another, as described above.

Step S58: The prediction unit 214 outputs the predicted reaction percentage by using the output device 30.

As described above, broadly speaking, the prediction device 20 of the present disclosure and the prediction method of the present disclosure execute two steps. In the first step, a prediction model is constructed by machine learning using, as training data, information on a plurality of monomers whose reaction percentage is known. In the second step, features are determined by molecular simulation (molecular calculation) from molecule information on the monomers, such as their molecular structures, rational formulae, etc., and the reaction percentage is predicted. Here, a prediction model constructed in advance may be used. Also, the prediction model may be updated as appropriate.

The prediction device 20 of the present disclosure and the prediction method of the present disclosure can predict the reaction percentage of reactive groups in formation of a resin by the reactive groups reacting with each other.

Also, in an example of the present disclosure, the reaction percentage prediction just requires inputting only molecule information on candidate monomers. Hence, it is not necessarily essential for the user to have expertise in resin materials. Thus, the designing of resin materials, which has previously been a task dependent on the expertise of experts in conventional technology, can now be efficiently promoted by a worker without expertise.

Also, the number of monomers to be used in the reaction (polymerization) can be predicted from the predicted reaction percentage. In this way, the number average molecular weight of a polymer chain that is the resin to be obtained can be predicted, and an average length of a polymer chain can be determined from the number average molecular weight. The number average molecular weight can be used to model the three-dimensional structure of the resin in molecular simulation.

Conventionally, when the three-dimensional structure of a resin is modeled, the length of a molecular chain is determined based on the operator's experience and know-how. Further, the reaction for the synthesis of the resin is simulated by molecular simulation to create a resin structure. Between these approaches, the former has a problem of decreased accuracy because the reaction percentage is not reflected in the model. On the other hand, the latter has a problem of requiring resources and time for calculation in the process of synthesizing the resin through molecular simulation. However, according to the present disclosure, it is possible to model a resin structure with higher accuracy in a shorter time.

### Reference Signs List

10 input device
100 prediction system
101 input screen
102 input field
20 prediction device
21 computation unit
211 molecular property determination unit (second determination unit)
212 reaction path determination unit (first determination unit)
213 prediction model construction unit
214 prediction unit
215 first construction unit
216 second construction unit
22 storage unit
222 database
223 molecular property DB (database, second indicator database)
224 reaction path DB (database, first indicator database)
225 prediction model DB (database)
226 first structure DB (database)
227 second structure DB (database)
23 reception unit
24 output unit
30 output device
601 phenomena
602 factors
603 features (second indicators)
604 header (identification information)
701 phenomena
702 features (first indicators)
703 reaction percentage
704 header (identification information)

## Claims

1. A prediction system comprising a prediction device which predicts a reaction percentage of reactive groups in a reaction between the reactive groups, the reactive groups included in monomers being molecules that form a resin, wherein
the prediction device includes
a database in which a first indicator of each of the monomers related to the activation energy defined by the Arrhenius equation, a second indicator of each of the monomers related to the frequency factor defined by the Arrhenius equation, and the reaction percentage are associated with one another, and
a prediction unit which predicts the reaction percentage from the first indicators and the second indicators of the monomers determined based on molecule information on the monomers, and from the database.

2. The prediction system according to claim 1, wherein the second indicator includes at least one of
a structure of the monomer corresponding to the molecule information, or
a molecular property being a physical property that is determined from an electronic state of the monomer corresponding to the molecule information and affect reactivity with another one of the monomers.

3. The prediction system according to claim 1 or 2, wherein
the database includes a prediction model which predicts the reaction percentage, and
the prediction system comprises a prediction model construction unit which constructs the prediction model by machine learning in which the first indicator and the second indicator are features and the reaction percentage is a response variable.

4. The prediction system according to claim 3, wherein the reaction percentage includes a measured value obtained by actually reacting the monomers with each other.

5. The prediction system according to claim 3, wherein the reaction percentage includes a calculated value obtained by molecular simulation assuming that the monomers are reacted with each other.

6. The prediction system according to claim 3, wherein the prediction model construction unit constructs a different prediction model as the prediction model for each of the resins.

7. The prediction system according to claim 1 or 2, further comprising a second determination unit which determines the second indicators to be used by the prediction unit by obtaining information corresponding to the molecule information from the database.

8. The prediction system according to claim 1 or 2, further comprising a first determination unit which determines the first indicators to be used by the prediction unit by obtaining information corresponding to the molecule information from the database.

9. The prediction system according to claim 8, wherein the first determination unit determines a structure of the resin from the monomers and determines a reaction path from the monomers to the resin by molecular simulation.

10. The prediction system according to claim 1 or 2, wherein the second indicator includes at least one of
at least one of an attraction or a motion exerted on at least one of the monomers or the reactive groups each of which is included in the monomer and reacts with another one of the monomers, or
steric hindrance of the reactive group against another one of the monomers.

11. The prediction system according to claim 1 or 2, wherein the first indicator includes at least one of
an activation energy of the monomer, or
heat of reaction in formation of the resin from the monomer.

12. The prediction system according to claim 1 or 2, wherein
the database includes a second indicator database including the second indicator, and
the prediction system comprises a second construction unit which organizes phenomena that occurs during the reaction between the reactive groups in chronological order and which identifies a factor that affects the phenomena to thereby construct the second indicator database including the second indicator as a molecular property representing the factor.

13. The prediction system according to claim 12, wherein the second construction unit constructs the second indicator database from the molecule information by at least one of descriptor calculation, quantum chemical calculation, molecular mechanics calculation, molecular dynamics calculation, first-principles calculation, or first-principles molecular dynamics calculation based on structures of the monomers.

14. The prediction system according to claim 12, wherein the second construction unit constructs the second indicator database by at least one of calculation of a molecular descriptor or execution of molecular simulation for a molecular structure of the resin obtained by arranging reactive functional groups forming the resin in a basic skeleton of the resin.

15. The prediction system according to claim 1 or 2, wherein the database includes identification information on types of at least one of the reactive groups or the resin being a product of the reaction between the reactive groups.

16. The prediction system according to claim 1 or 2, wherein
the database includes a first indicator database including the first indicator, and
the prediction system comprises a first construction unit which organizes phenomena that occurs during the reaction between the reactive groups in chronological order and which evaluates ease of occurrence of the phenomena to thereby construct the first indicator database.

17. The prediction system according to claim 16, wherein the first construction unit constructs the first indicator database by determining a three-dimensional structure of the resin the reaction percentage of which is known and monomers which form the resin, generating a plurality of three-dimensional structures in which reactive groups in the monomers to be subjected prediction of the reaction percentage are arranged close to each other, and performing structural optimization on the plurality of three-dimensional structures.

18. The prediction system according to claim 16, wherein if a reaction of forming the resin from the monomers progresses through a plurality of steps of reactions via an intermediate, the first construction unit constructs the first indicator database through a plurality of separate steps from the molecules to the intermediate and from the intermediate to the resin.

19. A prediction device comprising:
a database in which a first indicator of each of monomers being molecules that form a resin, a second indicator of each of the monomers, and a reaction percentage of reactive groups included in the monomers in a reaction between the reactive groups are associated with one another, the first indicator being related to the activation energy defined by the Arrhenius equation, the second indicator being related to the frequency factor defined by the Arrhenius equation; and
a prediction unit which predicts the reaction percentage from the first indicators and the second indicators of the monomers determined based on molecule information on the monomers, and from the database.

20. A prediction method comprising a prediction step of predicting a reaction percentage of reactive groups included in monomers in a reaction between the reactive groups from a database in which a first indicator of each of the monomers related to the activation energy defined by the Arrhenius equation, a second indicator of each of the monomers related to the frequency factor defined by the Arrhenius equation, and the reaction percentage are associated with one another, and from the first indicators and the second indicators of the monomers determined based on molecule information on the monomers, the monomers being molecules that form a resin.
